Europäisches Patentamt

European Patent Office

Office européen des brevets

(19)

(11) Veröffentlichungsnummer : **0 111 861**
**B1**

(12) # EUROPÄISCHE PATENTSCHRIFT

(45) Veröffentlichungstag der Patentschrift :
05.03.86

(51) Int. Cl.⁴ : **C 07 C 85/06**

(21) Anmeldenummer : **83112457.3**

(22) Anmeldetag : **10.12.83**

(54) **Verfahren zur Herstellung von Aminen.**

(30) Priorität : **18.12.82 DE 3246978**

(43) Veröffentlichungstag der Anmeldung :
**27.06.84 Patentblatt 84/26**

(45) Bekanntmachung des Hinweises auf die Patenterteilung : **05.03.86 Patentblatt 86/10**

(84) Benannte Vertragsstaaten :
**BE DE FR GB IT NL**

(56) Entgegenhaltungen :
**EP-A- 0 070 512**
**DE-A- 3 005 953**
**FR-A- 2 351 088**
**GB-A- 2 017 682**

(73) Patentinhaber : **BASF Aktiengesellschaft**
**Carl-Bosch-Strasse 38**
**D-6700 Ludwigshafen (DE)**

(72) Erfinder : **Mueller, Herbert, Dr.**
**Carostrasse 53**
**D-6710 Frankenthal (DE)**
Erfinder : **Axel, Hartmut, Dr.**
**Bahnhofsanlage 32**
**D-6830 Schwetzingen (DE)**

0 111 861

**Beschreibung**

Die vorliegende Erfindung betrifft ein neues Verfahren zur Herstellung von primären, sekundären oder tertiären Aminen durch Umsetzung von Ammoniak oder primären Aminen mit Alkoholen an kupferhaltigen Katalysatoren.

Bekannt ist die katalytische Alkylierung von Ammoniak oder Aminen mit Alkoholen, wobei dehydratisierende Oxide, z. B. die des Aluminiums, Thoriums, Wolframs oder Chroms als Katalysatoren verwendet werden. Auch Hydrierungs- bzw. Dehydrierungskatalysatoren mit den aktiven Metallen Kupfer, Nickel und Kobalt oder Edelmetallen sind für diesen Zweck empfohlen worden. Die Hydrierungs/ Dehydrierungskatalysatoren sind Feststoffe. Sie werden in Form einer Suspension, wenn sie in Pulverform vorliegen, oder als Formkörper im fest angeordneten Bett angewendet. Es wurden Verfahren in flüssiger Phase sowie in der Gasphase vorgeschlagen. In einer Arbeit von V.A. Nekrasova und N.I. Shuikin in der Zeitschrift Russian Chemical Reviews, *34*, 843 (1965) sowie in dem Buch « The acyclic aliphatic tertiary Amines », L. Spialter u. J.A. Pappalardo, The Macmillan Comp., 1965, ist eine ausführliche Darstellung des Sachgebietes abgehandelt.

Besonders schwierig ist die Herstellung von Aminen, bei denen unterschiedliche Alkylreste mit dem Stickstoffatom verknüpft sind. Als Folge von Transalkylierungsreaktionen bilden sich primäre, sekundäre und tertiäre Amine mit unerwünschter Alkylgruppenverteilung als Nebenprodukte, die oftmals nur mit sehr großem Aufwand von den gewünschten Reaktionsprodukten abzutrennen sind. Diese Transalkylierungsreaktionen treten besonders bei der Verwendung von Hydrierungs-/Dehydrierungskatalysatoren mit zu geringer Aktivität in den Vordergrund.

Die für die Alkylierung von Ammoniak oder primären Aminen mit Alkohol zu verwendenden Katalysatoren sind aus vielen Veröffentlichungen bekannt. Es handelt sich dabei in allen Fällen um metallische Katalysatoren, die entweder außerhalb des Reaktionssystems hergestellt und aktiviert oder zu Beginn der Katalyse durch Reduktion entwickelt werden.

Die US-PS-2 953 601 beschreibt dazu die Verwendung von Raney-Nickel oder von auf Aluminium niedergeschlagenem Nickel. Die Beispiele zeigen, daß Umsatz und Ausbeute dieser Reaktionen keine zufriedenstellenden Werte liefern.

Nach dem Vorschlag der US-PS-3 223 734 kann man Raney-Nickel, Kupfer-Chromoxid, Palladium auf Kohle oder Nickel auf Diatomeen-Erde als Katalysator zur Aminherstellung verwenden. Wie die Beispiele der Patentschrift Kg/IG zeigen, sind jedoch auch mit diesen Katalysatoren keine günstigen Ergebnisse zu erzielen, und es werden auch oft unwirtschaftlich große Mengen gebraucht.

Ein verbessertes Verfahren zur Herstellung von Aminen beschreibt die US-PS-3 708 539. Hier wird in flüssiger Phase ein Alkohol mit einem sekundären Amin an Ruthenium-, Osmium-, Rhenium- oder Technetium-Katalysatoren umgesetzt. Nachteilig bei diesem Verfahren ist vor allem der Umstand, daß Umsatz und Ausbeute, bezogen auf die eingesetzten Alkohole, gemessen am Wert der Katalysator-Rohstoffe, für ein technisches Verfahren unzureichend sind.

Eine wesentlich verbesserte Methode zur Herstellung von Dimethyldodecylamin wird in der JP-OS-19604/77 beschrieben. Man erhält Dimethyldodecylamin mit einem Kupfer-Chromoxid-Katalysator auf Diatomeen-Erde mit einer Ausbeute um 90 %.

Aus der DE-OS-22 55 701 ist ein Verfahren zur Herstellung von sekundären Aminen aus Alkohol und Ammoniak durch Suspensionskatalyse bekannt. Es zeigt sich, daß es bei dieser Reaktion sehr schwierig ist, die Bildung tertiärer Amine zu vermeiden. Nur wenn durch verhältnismäßig hohen technischen Aufwand ein Ammoniakunterschuß im Reaktionssystem vermieden wird, gelingt es, die sekundären Amine mit über 80 % Ausbeute herzustellen.

Deshalb wird in den DE-OSen-29 07 869 und 30 05 953 ein quasi-homogenes, kolloidales Katalysatorsystem vorgeschlagen, das die beschriebenen Nachteile der festen Katalysatoren nicht aufweist. Diese kolloidalen Katalysatoren entstehen bei der Reduktion einer Mischung carbonsaurer Salze von Kupfer oder Silber mit carbonsauren Salzen von Elementen der Gruppe VIII des Perioden-Systems (einschließlich Mangan und Zink) sowie carbonsauren Salzen der Alkali- und Erdalkali-Elemente mit Wasserstoff bzw. Aluminiumalkylverbindungen. Statt Salzen von Carbonsäuren können auch innere Komplexe von z. B. Dicarbonylverbindungen verwendet werden.

Bei diesen Katalysatoren erreicht man, wie auch sonst von Aminolysen allgemein bekannt, gute Aktivität und Selektivität nur dann, wenn eine Kombination aus mehreren aktiven Metallen in den richtigen Mischungsverhältnissen angewendet wird. Von Nachteil ist auch, daß die Katalysatoren vor der eigentlichen Umsetzung durch Reduktion mit Wasserstoff oder Aluminiumalkylen aktiviert werden müssen. Schließlich können die Katalysatoren nicht auf einfache Art und Weise mechanisch entfernt werden, sondern sollen durch Destillation des Reaktionsprodukts abgetrennt werden. Dadurch verbleibt der Katalysator in den gleichzeitig entstandenen höhersiedenden oder undestillierbaren Rückständen. Die Katalysatoren können zwar zunächst für eine weitere Umsetzung eingesetzt werden, es läßt sich aber nicht vermeiden, daß sich dadurch der Nebenproduktspiegel im Reaktionssystem mehr und mehr erhöht. Abgesehen davon, daß empfindliche Substanzen die Gegenwart katalytisch wirkender Metalle bei der Destillation schlecht überstehen und Veränderungen erleiden können, verbleibt noch das Problem, diese Katalysatoren zu einem späteren Zeitpunkt aus den Rückständen umweltfreundlich abzutrennen.

Es ist daher eine Aufgabe der Erfindung, aliphatische und cycloaliphatische Amine sowie Aralkylami-

2

# 0 111 861

ne auf einem Wege herzustellen, der eine hohe Ausbeute der Zielprodukte ermöglicht, wenig verunreinigende Nebenprodukte erzeugt und nur einen minimalen Katalysatoreinsatz erfordert. Der zu verwendende Katalysator soll je nach Wunsch die Herstellung von primären, sekundären oder tertiären Aminen ermöglichen. Außerdem soll eine möglichst vollständige Umsetzung der Alkohole eintreten, da die spätere Abtrennung der Alkohole von den Zielprodukten oftmals schwierig ist. Der Katalysator sollte schließlich nach der Umsetzung von einer solchen Beschaffenheit sein, daß er leicht quantitativ vom Reaktionsprodukt abgetrennt und für weitere Umsetzungen verwendet werden kann. Schließlich sollte die Bildung von Rückständen, höhermolekularen Kondensationsprodukten und Nebenreaktionen weitgehend ausgeschlossen werden. Dieser vielseitige Katalysator muß darüber hinaus aus einer einfachen, leicht zugänglichen Chemikalie entstehen und möglichst ohne einen eigenen Aktivierungsschritt unmittelbar für die Umsetzung wirksam werden.

Es wurde nun gefunden, daß die Herstellung von primären bzw. symmetrischen oder unsymmetrischen sekundären und tertiären Aminen mit insgesamt bis zu etwa 40 Kohlenstoffatomen durch Umsetzung von Ammoniak oder primären Aminen mit primären oder sekundären ein- oder mehrwertigen Alkoholen, insbesondere Alkoholen, die 6 oder mehr Kohlenstoffatome besitzen, an einem Kupferkatalysator bei Temperaturen von 170 bis 250 °C, gegebenenfalls in Gegenwart von Wasserstoff, vorteilhaft gelingt, wenn man als Katalysatorvorläufer Kupferformiat verwendet, wobei der Katalysator unter den Reaktionsbedingungen aus dem Kupferformiat von selbst entsteht.

Ein ähnliches Verfahren für die Herstellung tertiärer Amine, ausgehend von sekundären Aminen, ist Gegenstand der Anmeldung DE-OS-31 28 889.

Wie dort, geht man auch hier vorteilhaft in an sich bekannter Weise so vor, daß man Ammoniak oder das jeweilige primäre Amin zum flüssigen, den jeweiligen Alkohol enthaltenden Reaktionsgemisch im Maße der Umsetzung zuführt und Wasser im Maße seiner Entstehung entfernt.

Das erfindungsgemäße Verfahren wird hauptsächlich für die Umsetzung hochsiedender Alkohole mit Ammoniak bzw. den jeweiligen primären Aminen angewendet. (Handelt es sich um die Alkylierung eines hochsiedenden primären Amins mit einem tiefsiedenden Alkohol, dann ist es zweckmäßig, den Alkohol zum flüssigen, das Amin enthaltende Reaktionsgemisch im Maße der Umsetzung zuzuführen und Wasser im Maße seiner Entstehung zu entfernen.)

Dies kann beispielsweise so durchgeführt werden, daß man das Kupferformiat dem Alkohol (bzw. dem Amin) zusetzt und das Gemisch, gegebenenfalls in Gegenwart geringer Mengen von Amin (Alkohol), erhitzt. Bei einer Temperatur von ca. 170 °C setzt üblicherweise die beabsichtigte Umsetzung von selbst ein. Der Katalysator entfaltet unter diesen Bedingungen ohne vorhergehende zusätzliche Aktivierung seine maximale katalytische Wirkung.

Die zu alkylierenden Stickstoffkomponenten können mittels einer Dosiervorrichtung dem auf Reaktionstemperatur gebrachten Alkohol zugeführt werden. Diese Arbeitsweise wird bevorzugt, wenn die Reaktionstemperatur über dem Siedepunkt der einzusetzenden Aminkomponente liegt und die Reaktion isobar geführt wird. Man vermeidet so die Ansammlung größerer und überschüssiger Aminmengen, was sich vorteilhaft auf die Ausbeute auswirkt.

Bei absatzweiser Durchführung wird also üblicherweise die Reaktion unter allmählicher Zugabe des Ammoniaks oder des primären Amins bis zum Verbrauch des Alkohols geführt, wobei man dann das entstandene Amin durch Destillation des flüssigen Reaktionsgemisches erhalten kann.

In sinngemäßer Abwandlung wird man bei kontinuierlicher Betriebsweise Ammoniak oder primäres Amin beispielsweise gasförmig im Gleich- oder Gegenstrom zum flüssigen Reaktionsgemisch führen, das den Alkohol, den suspendierten katalysator sowie gegebenenfalls das bei der Reaktion entstandene Amin enthält. Gegebenenfalls sollte man hier eine Nachreaktionsstrecke vorsehen.

Eine wichtige Maßnahme zur Erzielung optimaler Ergebnisse besteht darin, das gebildete Wasser laufend aus dem Reaktionsgemisch zu entfernen, woraus sich ergibt, daß man vorteilhaft unter Bedingungen arbeitet, bei denen Wasser freiwillig als Gas das Reaktionsgemisch verläßt.

Zum Verständnis der Erfindung kann man sich folgendes vorstellen : findet die Reaktion in der Gasphase statt, so muß eine sehr hohe Reaktionstemperatur gewählt werden, da die Alkohole im allgemeinen relativ schwer flüchtig sind ; man erhält dann eine geringe Ausbeute und unreine Produkte. Wird andererseits in flüssiger Phase gearbeitet, indem man z. B. — bei leicht flüchtigen Aminen wie Ethylamin — hohen Druck unter Berücksichtigung der Temperatur und eine hohe Konzentration an Amin anwendet, so ist der Reaktionsverlauf in hohem Maße unspezifisch. In diesem Sinne wirkt auch die mit dem Umsatz gewöhnlich zunehmende Wassermenge.

Zweckmäßig hält man die Reaktionsbedingungen ein, wie sie in der DE-OS-26 25 196 beschrieben werden. Durch die Anwendung von Kupferformiat als Katalysator gelingt es dabei mit wesentlich geringeren Katalysatormengen auszukommen, reinere Produkte zu erhalten und die Umsetzung auch zur Herstellung von sekundären Aminen anzuwenden. Zugleich werden auch höhere Ausbeuten erzielt.

Vorteilhaft ist es weiterhin, in Gegenwart von z. B. 0,1 bis 50 Gew.-%, vorzugsweise 1 bis 10 Gew.-% (bezogen auf den Kupferkatalysator) einer Base, etwa Alkali- oder Erdalkalihydroxid, z. B. Natriumhydroxid, Kaliumhydroxid, Calciumhydroxid sowie besonders vorteilhaft den entsprechenden Carbonaten zu arbeiten, wobei mehrere der besagten Verbindungen gleichzeitig anwesend sein können.

Die Reaktion kann an sich — entsprechend der Brutto-Umsatzgleichung — in Abwesenheit von Wasserstoff durchgeführt werden. So schreitet die Reaktion beispielsweise zwischen einem Alkohol und Ammoniak

3

allein durch Zuführung des Ammoniakgases zur Reaktionsmischung fort, sobald die notwendige Reaktionstemperatur erreicht ist. Man erhält das gewünschte Amin in einer Ausbeute von 90 bis 95 % bei einer Alkoholumwandlung von über 99 %. Dennoch hat es sich in manchen Fällen als vorteilhaft erwiesen, geringe Mengen Wasserstoff dem Reaktionsmilieu zuzuführen, was der Aktivität des Katalysators unter Umständen förderlich ist und verhindert, daß geringe Mengen ungesättigte Verbindungen entstehen. Arbeitet man jedoch in Abwesenheit von Wasserstoff und es bilden sich dadurch geringe Mengen ungesättigter Verbindungen, so empfiehlt es sich, diese nach Beendigung der Aminierung zu hydrieren.

An die Beschaffenheit und die Eigenschaften des Kupferformiats werden keine besonderen Anforderungen gestellt. Man kann z. B. handelsübliches kristallwasserhaltiges Kupferformiat ohne Vorbehandlung einsetzen. Mit demselben Erfolg läßt sich aber auch wasserfreies Kupferformiat verwenden. Die Art und Weise, wie das Kupfersalz hergestellt wurde, ist für dessen katalytische Aktivität ohne Bedeutung. Beispielsweise erhält man einen geeigneten Katalysator, wenn man das Formiat aus dem Kupferoxid, -hydroxid, -stearat oder -carbonat und Ameisensäure herstellt. Diese Umsetzung kann auch in Abwesenheit von Wasser, z. B. in dem für die Aminolyse vorgesehenen Alkohol mit der Kupferverbindung und Ameisensäure erfolgen. Schließlich kann man auch einfach wäßrige Kupferformiat-Lösung als Katalysatorvorläufer dem Alkohol zumischen. Das ins Reaktionsmilieu eingetragene Wasser destilliert ab, noch ehe die Reaktionstemperatur beim Aufheizen erreicht wird. Es kann angenommen werden, daß sich der eigentliche Katalysator durch die Umsetzung von Kupferformiat mit Bestandteilen des Reaktionsgemisches von selbst bildet. Die Menge an Kupferformiat zur Erzielung eines ausreichenden Umsatzes und eine genügend hohen Reaktionsgeschwindigkeit beträgt 0,01 bis 2 Gew.-%, vorzugsweise 0,05 bis 1 Gew.-% (berechnet als metallisches Kupfer), bezogen auf die Summe der Einsatzstoffe. Da sich die aus dem Kupferformiat entstehenden Katalysatoren bei Verwendung von reinen Ausgangsmaterialien praktisch nicht verbrauchen und immer wieder verwendet werden können, spielen dessen geringe Kosten keine Rolle. So können die Katalysatoren irgendwann abgetrennt und wieder in Kupferformiat umgewandelt und gegebenenfalls nach erneuter Zugabe einer basischen Alkali- oder Erdalkalimetallverbindung wiederum verwendet werden.

Der als Ausgangsmaterial verwendete Alkohol kann einen linearen oder verzweigten, gesättigten oder ungesättigten, aliphatischen Kohlenwasserstoffrest besitzen, dessen Kohlenstoffkette durch Heteroatome, insbesondere Sauerstoffatome unterbrochen sein kann. Obwohl auch sekundäre Alkohole nach dem vorliegenden Verfahren umgesetzt werden können, wird die Reaktion mit primären Alkoholen bevorzugt. Die besten Ergebnisse werden also mit mono- und polyfunktionellen primären Alkoholen erhalten, deren Anzahl an Kohlenstoffatomen 2 oder mehr beträgt.

Technisch besonders wichtige Zielprodukte sind die sogenannten Fettamine ; sie leiten sich von Alkoholen ab, die etwa 6 bis 22 Kohlenstoffatome im Molekül aufweisen (sogenannte Fettalkohole). Genannt seien Octylalkohol, Laurylalkohol, Myristylalkohol, Cetylalkohol, Oleylalkohol, Stearylalkohol, Tridecylalkohol, Pentadecylalkohol oder Gemische dieser Verbindungen.

Ferner eignen sich z. B. zweiwertige Alkohole wie Ethylenglykol, Diethylenglykol, Butandiol-1,4, Hexandiol-1,6.

Alkohole, deren Anzahl an Kohlenstoffatomen < 6 ist, werden nach dem erfindungsgemäßen Verfahren hauptsächlich für die Herstellung von unsymmetrischen sekundären oder tertiären Aminen verwendet ; dabei sollte die Anzahl der Kohlenstoffatome des als Ausgangsprodukt verwendeten primären Amins ≥ 8 sein (d. h. schwerflüchtige Amine). Ferner besteht bei diesen niederen Alkoholen die Möglichkeit, die entsprechenden mehrwertigen Alkohole einzusetzen, die schwerflüchtig sind.

Anstelle der Alkohole können auch lineare und verzweigte, gesättigte und ungesättigte, aliphatische Aldehyde, wie Laurylaldehyd oder auch Oxoaldehyde verwendet werden, die in Gegenwart von Wasserstoff unter den Reaktionsbedingungen zu Alkoholen reduziert werden.

Primäre Amine, die — neben Ammoniak — für das erfindungsgemäße Verfahren geeignet sind, besitzen Alkyl-, Cycloalkyl- oder Aralkylsubstituenten mit einer Kohlenstoffzahl von z. B. 1 bis 18 oder mehr, vorzugsweise 1 bis 16 und insbesondere 1 bis 12. Technisch wichtige primäre Amine sind beispielsweise Methylamin, Propylamin, Octylamin, Decylamin oder Laurylamin.

Die Reaktion findet bei einer Temperatur zwischen i. a. 170 und 250 °C, vorzugsweise zwischen 180 und 220 °C statt. Sie kann unter atmosphärischem, erhöhtem oder erniedrigtem Druck ausgeführt werden, beispielsweise in einem Bereich von 10 mbar bis 6 bar, wobei der zu wählende Druckbereich auch von den Eigenschaften der beteiligten Reaktionspartner und der gewählten Reaktionstemperatur abhängt.

Verwendet man für die Alkylierung einen hochsiedenden Alkohol, beispielsweise Decanol, Laurylalkohol oder Tridecylalkohol, so genügt es, den Alkohol in Gegenwart des Kupferformiats und gegebenenfalls unter Zusatz der aktivierenden Alkaliverbindung zu erhitzen und Ammoniak oder das primäre Amin im Maße wie es reagiert, einzuleiten. Das bei der Umsetzung gebildete Wasser wird aus dem Reaktionssystem laufend abdestilliert. Dieser letzte Vorgang kann dadurch gefördert werden, daß man mit einem geeigneten Lösungsmittel, etwa einem aliphatischen oder aromatischen Kohlenwasserstoff, das Reaktionswasser als Azeotrop auskreist und abtrennt. Als Reaktionsraum ist deshalb z. B. ein Reaktor geeignet, der mit einer Rührvorrichtung, einem Kondensator und einem Wasserabtrenngefäß ausgerüstet ist. Eine besonders einfache Methode besteht nun darin, daß man dem auf Reaktionstemperatur gebrachten Alkohol Ammoniak oder das primäre Amin und falls gewollt Wasserstoff zuführt. Dabei wird das entstandene Wasser dampfförmig aus der Reaktionszone entfernt und kondensiert und das unverbrauchte Gas wieder zurückgeleitet. Je nach Reaktionstemperatur und Menge des Katalysators kann man stündlich bis zur äquimolaren Menge, vorzugsweise 0,3 bis 0,6 Mol des Amins pro Mol Alkohol in das Reaktionsgemisch einleiten. Die gegebenenfalls zu verwendende Wasserstoffmenge liegt entweder in der gleichen Größenordnung oder auch erheblich darunter. Sie ist auf jeden Fall in weiten Grenzen variabel.

Aus dem vorstehenden erkennt man, daß man günstige Reaktionsbedingungen dann gewählt hat, wenn es gelingt, die Umsetzung etwa in 1 bis 10 Stunden auszuführen. Kürzere Reaktionszeiten sind wegen der aufzubringenden Wärmemenge für das zu verdampfende Reaktionswasser nicht zu empfehlen. Dagegen können längere Reaktionszeiten ohne Nachteil für die Umsetzung in Kauf genommen werden. Die Reaktionsgeschwindigkeit wird darüber hinaus natürlich immer von der pro Zeiteinheit zugegebenen Menge an Amin bestimmt. Normalerweise wird man sie so wählen, daß die Konzentration an Ammoniak oder an primärem Amin gleichbleibend wenige Prozent des Reaktionsgemisches beträgt, d. h. im zeitlichen Mittel wird bevorzugt der Alkohol in großem Überschuß im Vergleich zum Amin eingesetzt.

Ein besonderes Ergebnis der Erfindung ist es, daß die Reaktionsprodukte nach vollständigem Umsatz und mechanischer Abtrennung des Katalysators üblicherweise ohne weitere Reinigungsoperation für viele Einsatzgebiete eine genügende Reinheit aufweisen. Die Produkte sind durchweg farblos und sind meist mit weniger als 5 Gew.% Nebenprodukten verunreinigt.

Dies bedeutet insbesondere bei der Herstellung hochsiedender tertiärer Amine (z. B. Trioctylamin, das als Extraktionsmittel für Erze dient) den Wegfall der destillativen Reinigung und damit eine nicht zu vernachlässigende Energieeinsparung. Gegenüber der Verfahrensweise, wie sie in der DE-OS-29 07 869 beschrieben ist, ist außer den bereits mitgeteilten Vorteilen zu erwähnen, daß die Alkylierung bei merklich tieferen Temperaturen einsetzt. Im Durchschnitt liegt die Reaktionstemperatur im erfindungsgemäßen Verfahren 10 bis 20 °C niedriger. Nicht zuletzt dadurch erklärt sich übrigens die nach dem erfindungsgemäßen Verfahren erzielte hohe Selektivität.

Besonders überraschend ist die hohe Selektivität bei der Herstellung von sekundären Aminen, ausgehend von Ammoniak, denn es ist bekannt, daß sich Alkylamine bei der Umsetzung mit Alkoholen sehr viel reaktiver verhalten als Ammoniak (Process Evaluation and Research Planning Service, Report 1978, Hrsg. Chem. System, New York, S. 429, letzter Abschnitt).

Da es unter anderem ein Ziel des erfindungsgemäßen Verfahren ist, den Alkohol vollständig umzusetzen, wird man Ammoniak oder das primäre Amin insgesamt mindestens in stöchiometrischer Menge anbieten. Von einem leichtflüchtigen Amin oder Ammoniak kann ein gewisser Überschuß gegen Reaktionsende zweckmäßig sein. Dieser mag im Durchschnitt zwischen 5 und 50 Mol.-%, bevorzugt 10 bis 20 Mol.-% betragen, d. h. man hält gegebenenfalls die Umsatzbedingungen so lange ein, bis ein entsprechender Umsatz erwartet werden kann. Überschüssig angebotener Ammoniak oder primäres Amin wird dann wieder entfernt. Es ist wichtig, daß Ammoniak oder primäres Amin im Verlaufe der Umsetzung in dem Maße zugegeben werden, wie sie sich umsetzen. Dadurch wird erfindungsgemäß erreicht, daß während der gesamten Reaktionsdauer der Alkohol im Überschuß vorliegt, d. h. solange dieser noch in nennenswerter Konzentration im Reaktionsgemisch vorhanden ist. Solche Reaktionsbedingungen sind sehr einfach bei einem Gegenstromverfahren zu erreichen, das darüber hinaus in fortlaufender Betriebsweise ausgeübt werden kann.

Die folgenden Beispiele sollen die Erfindung erläutern :

Beispiel 1

In einer Rührapparatur wurden bei Normaldruck 5 200 kg n-Octan-l-ol, 100 kg Kupferformiat $(Cu(HCOO)_2) \cdot 4H_2O)$, 500 kg N-Octylamin und 10 kg Calciumhydroxid vorgelegt und unter Zuleitung von 10 m³ Wasserstoff/Stunde auf 180 °C erhitzt. Es wurde 1 Stunde bei dieser Temperatur gerührt ; anschließend erhöhteman die Temperatur auf 200 °C und führte dabei gleichzeitig Ammoniak zu.

Im Verlauf von 10 Stunden wurden dem Reaktionssystem nun 240 kg Ammoniak zugesetzt, wobei ca. 5 % dieser Menge als Abgas abgeführt wurden.

Während in der Anfangsphase ca. 70 kg/Stunde Ammoniak umgesetzt wurden, sank diese Umsetzungsrate gegen Ende der Reaktion auf weniger als 1 kg/Stunde. Nach Abtrennen einer repräsentativen Probe stellte man durch Destillation und quantitative Gaschromatographie, bezogen auf den eingesetzten Alkohol, folgende Produktausbeuten fest :

| | |
|---|---|
| Tri-n-octylamin | 94 % d. Th. |
| Di-n-octylamin | 2 % d. Th. |
| n-Octylamin | 1 % d. Th. |
| n-Octan-l-ol | 0,5 % d. Th. |

(Das anfänglich zugesetzte Octylamin wurde bei der Berechnung der Ausbeuten berücksichtigt.)
Es blieb ein Destillationsrückstand von 2 Gew.-%, bezogen auf den eingesetzten Alkohol.
Der Katalysator wurde durch Zentrifugieren aus dem Reaktionsansatz vollständig abgetrennt und in den Rührreaktor zurückgebracht, wo er für erneute Umsetzungen verwendet wurde. Dazu wurden vor jedem neuen Ansatz 5 kg Kupferformiat und 1 kg Calciumhydroxid im Rührbehälter ergänzt und dann die Aminierung wie oben beschrieben durchgeführt. Auf diese Weise ließen sich 10 Aminierungen ausführen, ohne daß ein Abfall der Reaktionsgeschwindigkeit oder der Selektivität bemerkbar war.

Bei Durchführung der Reaktion ohne den Zusatz von Calciumhydroxid benötigte man für den vollständigen Alkoholumsatz eine Reaktionszeit von ca. 40 Stunden. Die Ausbeute an Tri-n-octylamin lag aber nur bei 88 bis 90 % d. Th., bezogen auf die eingesetzte Alkoholmenge.

Bei Ersatz von Calciumhydroxid durch Bariumhydroxid oder Kaliumcarbonat erhielt man Tri-n-octylamin in ca. 93 % d. Th. Während man durch den Zusatz von Calciumoxid ein geringfügig schlechteres Ergebnis erzielte, wurde durch die Dotierung des Katalysators mit Natriumcarbonat oder Natriumbicarbonat eine um ca. 20 % erhöhte Reaktionsgeschwindigkeit erreicht.

## Beispiel 2

In einer Rührapparatur wurden bei Normaldruck 8 000 kg n-Decan-l-ol, 360 kg Kupferformiat und 40 kg Calciumhydroxid vorgelegt und auf 180 °C erwärmt.

Gleichzeitig wurde ein Gemisch, bestehend aus 6 Vol.-Teilen Ammoniak und 1 Vol.-Teil Wasserstoff, unter die Oberfläche der Reaktionsmischung geleitet. Bei 180 °C wurden dem Gemisch im Verlauf von 13 Stunden auf diese Weise 480 kg Ammoniak zugesetzt, wobei ca. 10 % dieser Menge als Abgas abgeführt wurden.

Nach dieser Zeit war die gleichzeitig erfolgende Wasserabscheidung praktisch beendet. Man hielt das Reaktionsgemisch, ohne Zuführung von Ammoniak und Wasserstoff, noch 3 Stunden bei obiger Temperatur. Nach dem Abkühlen trennte man vom Katalysator ab und destillierte das klare Reaktionsgemisch, dessen quantitative GC-Analyse ergab, daß auf diese Weise vom eingesetzten n-Decan-l-ol

88 bis 90 Mol.-% in Di-n-decylamin
10 bis 12 Mol.-% in Tri-n-decylamin und
ca. 2 Mol.-% in n-Decylamin

übergeführt wurden.

Es blieb ein Destillationsrückstand von 1 Gew.-%, bezogen auf den eingesetzten Alkohol.

## Beispiel 3

In einer Rührapparatur wurden bei Normaldruck 8 000 kg n-Decan-l-ol, 200 kg Kupferformiat und 50 kg Calciumhydroxid vorgelegt. Unter Einleitung eines Gemisches, das anfänglich aus 8 Vol.-Teilen Methylamin und 1 Vol.-Teil Wasserstoff bestand, wurde das Reaktionssystem rasch auf 200 °C erwärmt. Während dann die eingeleitete Wasserstoffmenge über die gesamte Reaktionszeit konstant bei 5 m$^3$/h gehalten wurde, setzte man soviel Methylamin zu, daß die gesamte Menge gerade umgesetzt und ca. 10 % davon als Abgas abgeführt wurden.

Auf diese Weise wurden innerhalb von 17 Stunden 1 300 kg Methylamin eingeleitet.

Da die Aminzahl des Reaktionsgemisches bei 236 mg KOH/g lag, mußten weitere 2 550 kg n-Decan-l-ol hinzugefügt werden. Dann rührte man noch 8 Stunden unter Wasserstoffbegasung bei obiger Temperatur nach. Nach dem Abkühlen trennte man vom Katalysator ab und arbeitete destillativ auf.

Das Rohprodukt, dessen Aminzahl nun bei 172 mg KOH/g (berechneter Wert für Methyldi-n-decylamin 180 mg KOH/g) lag, setzte sich wie folgt zusammen :

| | |
|---|---|
| Methyldi-n-decylamin | 93 Gew.-% |
| Methyl-n-decylamin | 3 Gew.-% |
| n-Decan-l-ol | 0,1 Gew.-% |
| nichtflüchtige Bestandteile | 4 Gew.-% |

## Beispiel 4

150 kg 2-Hydroxydecahydronaphthalin, 3 kg Kupferformiat und 2 kg Calciumhydroxid wurden 4 Stunden bei 190 °C mit überschüssigem Ammoniak und Wasserstoff (Volumenverhältnis 8 : 1) begast. Danach wurde die Ammoniakzufuhr beendet und bei Reaktionstemperatur für weitere 2 Stunden nur noch mit Wasserstoff begast. Die Aufarbeitung erfolgte wie üblich. Man erhielt Bis(decahydronaphthyl)amin in einer Ausbeute von 98 % d. Th.

## Beispiel 5

Der Katalysator, der nach der Durchführung der Reaktionen, wie sie im Beispiel 1 beschrieben wurden, verblieb, wurde durch Waschen mit Tetrahydrofuran vom anhaftenden Amin befreit und zur Herstellung von Butyldodecylamin eingesetzt.

5 000 kg Dodecylamin wurden in der in Beispiel 1 beschriebenen Apparatur zusammen mit dem Katalysator auf 190 bis 200 °C erhitzt und stündlich mit ca. 200 kg Butanal und 10 m$^3$ Wasserstoff versetzt. Das bei der Alkylierung entstehende Reaktionswasser und überschüssiges Butanal passierten einen bei 100 °C betriebenen Dephlegmator und wurden anschließend kondensiert, wobei sich das Kondensat in zwei Phasen trennte. Die untere wäßrige Phase wurde verworfen, während die obere Phase, die im wesentlichen aus Butanal bestand, in die Reaktion zurückgeführt wurde.

Nach 10 Stunden fiel kein Reaktionswasser mehr an, die Reaktion wurde unterbrochen und das Reaktionsprodukt abgekühlt. Das Reaktionsprodukt enthielt nach GC-Analyse :

| | |
|---|---|
| Butyldodecylamin | 80 Gew. % |
| Dibutyldodecylamin | 11,5 Gew. % |
| Ausgangsprodukte | 1 bis 1,5 Gew. % |
| höhersiedende Anteile | 5 bis 7 Gew. % |

Die Endprodukte lassen sich durch Destillation rein gewinnen.

**Patentansprüche**

1. Verfahren zur Herstellung von primären bzw. symmetrischen oder unsymmetrischen sekundären und tertiären Aminen mit insgesamt bis zu etwa 40 Kohlenstoffatomen durch Umsetzung von Ammoniak oder primären Aminen mit primären oder sekundären ein- oder mehrwertigen Alkoholen, insbesondere Alkoholen, die 6 oder mehr Kohlenstoffatome besitzen, an einem Kupferkatalysator bei Temperaturen von 170 bis 250 °C, gegebenenfalls in Gegenwart von Wasserstoff, dadurch gekennzeichnet, daß man als Katalysatorvorläufer Kupferformiat verwendet, wobei der Katalysator unter den Reaktionsbedingungen aus dem Kupferformiat von selbst entsteht.

2. Verfahren nach Anspruch 1, dadurch gekennzeichnet, daß man die Umsetzung in Gegenwart einer basischen Alkali- oder Erdalkalimetallverbindung vornimmt.

3. Verfahren nach Anspruch 1, dadurch gekennzeichnet, daß man Ammoniak oder ein primäres Amin dem flüssigen, den Alkohol enthaltenden Reaktionsgemisch im Maße der Umsetzung zuführt und Wasser nach seiner Entstehung entfernt.

4. Verfahren nach Anspruch 1, dadurch gekennzeichnet, daß man Ammoniak oder ein leichtflüchtiges primäres Amin in Dampfform zuführt.

5. Verfahren nach Anspruch 1, dadurch gekennzeichnet, daß man Ammoniak oder ein primäres Amin bei einer Temperatur, die oberhalb der jeweiligen Siedetemperatur dieser Aminokomponenten liegt, dem Reaktionsgemisch zuführt und die Reaktion bei konstantem Druck ablaufen läßt.

6. Verfahren nach Anspruch 1, dadurch gekennzeichnet, daß man die Umsetzung bei absatzweiser Durchführung jeweils bis zum Verbrauch des Alkohols führt und aus dem flüssigen Reaktionsgemisch das entstandene Amin durch Destillation gewinnt.

7. Verfahren nach Anspruch 1, dadurch gekennzeichnet, daß man bei fortlaufender Betriebsweise Ammoniak oder ein leichtflüchtiges primäres Amin gasförmig im Gegenstrom zu dem Alkohol und gegebenenfalls gebildetes Amin enthaltenden flüssigen Reaktionsgemisch führt und gegebenenfalls eine Nachreaktionsstrecke vorsieht.

8. Verfahren nach Anspruch 1, dadurch gekennzeichnet, daß der vom Reaktionsprodukt abgetrennte Katalysator für weitere Umsetzungen, gegebenenfalls nach erneuter Zugabe einer basischen Alkali- oder Erdalkalimetallverbindung, erneut verwendet wird.

**Claims**

1. A process for the preparation of a primary or symmetric or asymmetric secondary or tertiary amine with a total of not more than about 40 carbon atoms by reacting ammonia or a primary amine with a primary or secondary monohydric or polyhydric alcohol, in particular an alcohol of 6 or more carbon atoms, over a copper catalyst at from 170 to 250 °C in the presence or absence of hydrogen, wherein copper formate is used as catalyst precursor, the catalyst forming of its own accord from the copper formate under the reaction conditions.

2. A process as claimed in claim 1, wherein the reaction is carried out in the presence of a basic alkali metal or alkaline earth metal compound.

3. A process as claimed in claim 1, wherein ammonia or a primary amine is added to the liquid reaction mixture containing the alcohol, at a rate corresponding to the reaction, and water is removed as it forms.

4. A process as claimed in claim 1, wherein ammonia or a primary amine is added in vaporous form.

5. A process as claimed in claim 1, wherein ammonia or a primary amine is added to the reaction mixture at a temperature above the particular boiling point of this amino component, and the reaction is carried out under constant pressure.

6. A process as claimed in claim 1, wherein, in the case of a batchwise procedure, the reaction is in each case carried out until the alcohol has been consumed, and the amine formed is isolated from the liquid reaction mixture by distillation.

7. A process as claimed in claim 1, wherein, in the case of a continuous procedure, ammonia or a highly volatile primary amine is passed in gaseous form countercurrently to the liquid reaction mixture containing the alcohol and any amine formed, and. if desired, an after-reaction zone is provided.

8. A process as claimed in claim 1, wherein the catalyst which has been separated off from the reaction product is used again for further reactions after the optional renewed addition of a basic alkali metal or alkaline earth metal compound.

**Revendications**

1. Procédé pour la préparation d'amines primaires ou secondaires symétriques ou dissymétriques et tertiaires contenant au total jusqu'à 40 atomes de carbone environ, par réaction d'ammoniac ou d'amines primaires avec des mono-alcools ou des poly-alcools primaires ou secondaires qui comportent 6 atomes de carbone ou plus, en présence de catalyseurs au cuivre, à des températures de 170 à 250 °C et, le cas échéant, en présence d'hydrogène, caractérisé en ce qu'on utilise du formiate de cuivre comme progéniteur de catalyseur, le catalyseur se formant de lui-même à partir du formiate de cuivre dans les conditions de la réaction.

2. Procédé selon la revendication 1, caractérisé en ce qu'on procède à la réaction en présence d'un composé basique de métal alcalin ou alcalinoterreux.

3. Procédé selon la revendication 1, caractérisé en ce qu'on envoie l'ammoniac ou une amine primaire dans. le mélange réactionnel liquide qui contient l'alcool dans la mesure de la réaction et on élimine l'eau à la suite de sa formation.

4. Procédé selon la revendication 1, caractérisé en ce qu'on introduit sous forme de vapeur l'ammoniac ou une amine primaire très volatile.

5. Procédé selon la revendication 1, caractérisé en ce qu'on envoie dans le mélange réactionnel l'ammoniac ou une amine primaire à une température qui se situe au-dessus du point d'ébullition particulier de ces composants aminés et on laisse la réaction se dérouler sous pression constante.

6. Procédé selon la revendication 1, caractérisé en ce qu'en cas de mode opératoire par charges successives, on conduit la réaction jusqu'à la consommation de l'alcool et on récupère l'amine formée, dans le mélange réactionnel liquide, par distillation.

7. Procédé selon la revendication 1, caractérisé en ce qu'en cas de mode opératoire en continu, on fait passer l'ammoniac ou une amine primaire très volatile sous forme de gaz à contre-courant de l'alcool et du mélange réactionnel liquide contenant l'amine éventuellement formée et, le cas échéant, on prévoit un trajet de réaction subséquent.

8. Procédé selon la revendication 1, caractérisé en ce que le catalyseur séparé du produit de la réaction est utilisé de nouveau pour d'autres réactions, le cas échéant après une nouvelle addition d'un composé basique de métal alcalin ou alcalinoterreux.